Europäisches Patentamt
European Patent Office
Office européen des brevets

(11) Veröffentlichungsnummer: **0 348 735 A2**

# EUROPÄISCHE PATENTANMELDUNG

(21) Anmeldenummer: 89110797.1

(22) Anmeldetag: 14.06.89

(51) Int. Cl.4: C07D 277/56 , A01N 43/78 , C07D 277/32

(30) Priorität: 27.06.88 DE 3821598

(43) Veröffentlichungstag der Anmeldung: 03.01.90 Patentblatt 90/01

(84) Benannte Vertragsstaaten: BE CH DE FR GB IT LI NL

(71) Anmelder: BAYER AG
D-5090 Leverkusen 1 Bayerwerk(DE)

(72) Erfinder: Förster, Heinz, Dr.
Am Eckbusch 47
D-5600 Wuppertal 1(DE)
Erfinder: Beck, Gunther, Dr.
Am Mittelberg 19
D-5090 Leverkusen 1(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch Gladbach 2(DE)
Erfinder: Strang, Harry, Dr.
Unterdorfstrasse 6A
D-4000 Düsseldorf 31(DE)

(54) **5-Chlor-4-cyano-thiazol-2-yl-oxyessigsäureamide.**

(57) Die Erfindung betrifft neue 5-Chlor-4-cyano-thiazol-2-yl-oxyessigsäureamide, ein Verfahren und neue Zwischenprodukte zu ihrer Herstellung sowie ihre Verwendung als Herbizide. Die neuen Herbizidwirkstoffe entsprechen der Formel (I)

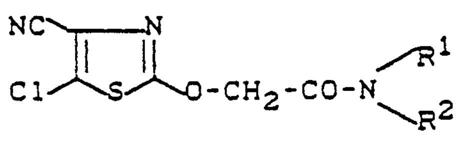

(I)

in welcher
$R^7$ für Wasserstoff oder für einen gegebenenfalls substituierten Rest aus der Reihe Alkyl, Alkenyl, Alkinyl oder Aralkyl steht,
$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
$R^1$ und $R^2$ zusammen mit dem Stickstoffatom, an welches sie gebunden sind, einen gegebenenfalls substituierten, gesättigten oder ungesättigten Heterocyclus bilden, der weitere Heteroatome enthalten kann und an den eine Benzo-Gruppierung anelliert sein kann.

EP 0 348 735 A2

## 5-Chlor-4-cyano-thiazol-2-yl-oxyessigsäureamide

Die Erfindung betrifft neue 5-Chlor-4-cyano-thiazol-2-yl-oxyessigsäureamide, ein Verfahren und neue Zwischenprodukte zu ihrer Herstellung sowie ihre Verwendung als Herbizide.

Es ist bereits bekannt, daß bestimmte Heteroaryloxyessigsäureamide, wie z. B. 2-(Benzthiazol-2-yl-oxy)-essigsäure-N-methyl-anilid, herbizide Eigenschaften aufweisen (vgl. US-P 4 509 971). Ferner sind auch bestimmte 4-Chlor- und 4-Methyl-5-cyano-thiazol-2-yl-oxyessigsäureamide als Herbizide bekanntgeworden (vgl. DE-A-3 038 608).

Die herbizide Wirksamkeit der vorbekannten Verbindungen ist jedoch nicht immer ganz zufriedenstellend.

Es wurden nun neue 5-Chlor-4-cyano-thiazol-2-yl-oxyessigsäureamide der allgemeinen Formel (I)

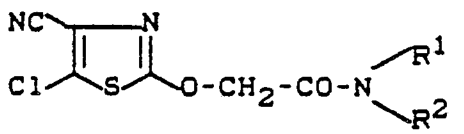

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Heterocyclus bilden, der weitere Heteroatome enthalten kann und an den eine Benzo-Gruppierung anelliert sein kann,

gefunden.

Weiter wurde gefunden, daß man die neuen 5-Chlor-4-cyano-thiazol-2-yl-oxyessigsäureamide der allgemeinen Formel (I) erhält, wenn man 4-Cyano-2,5-dichlor-thiazol der Formel (II)

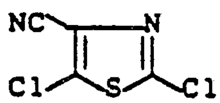

(II)

mit Hydroxyessigsäureamiden der allgemeinen Formel (III)

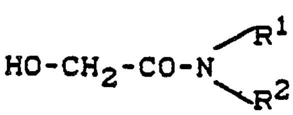

(III)

in welcher

$R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen 5-Chlor-4-cyano-thiazol-2-yl-oxyessigsäureamide der allgemeinen Formel (I) interessante herbizide Eigenschaften besitzen.

Überraschenderweise zeigen die neuen 5-Chlor-4-cyanothiazol-2-yl-oxyessigsäureamide der allgemeinen Formel (I) erheblich stärkere herbizide Wirkung gegen verbreitete, schwer bekämpfbare Unkräuter als die oben genannte Verbindung bei guter Verträglichkeit gegenüber wichtigen Kulturpflanzen.

Gegenstand der Erfindung sind vorzugsweise Verbindungen der Formel (I), in welcher

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für

$C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

Die Erfindung betrifft insbesondere Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy, Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydro-chinolinyl stehen.

Verwendet man 4-Cyano-2,5-dichlor-thiazol und Hydroxyessigsäurepiperidid als Ausgangstoffe, so läßt sich der Reaktionsablauf beim erfindungsgemäßen Verfahren durch das folgende Formelschema darstellen:

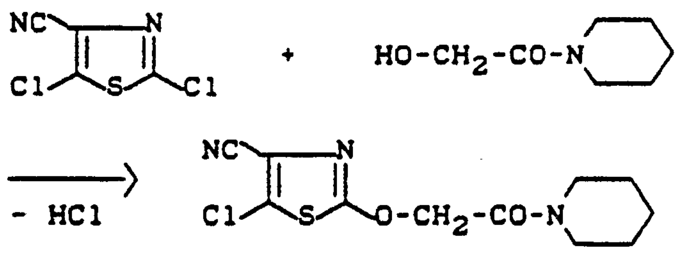


Das als Ausgangsverbindung zu verwendende 4-Cyano-2,5-dichlor-thiazol der Formel (II) ist noch nicht aus der Literatur bekannt und ist Gegenstand der vorliegenden Erfindung.

Man erhält die neue Verbindung der Formel (II), wenn man 4-Carbamoyl-2,5-dichlor-thiazol der Formel (IV)

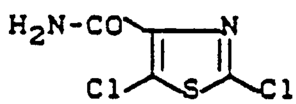

(IV)

mit einem Dehydratisierungsmittel, wie z. B. Phosphoroxychlorid, bei Temperaturen zwischen 50 °C und 150 °C umsetzt.

Das als Zwischenprodukt benötigte 4-Carbamoyl-2,5-dichlor-thiazol der Formel (IV) ist ebenfalls noch nicht aus der Literatur bekannt und Gegenstand der vorliegenden Erfindung.

Man erhält die neue Verbindung der Formel (IV), wenn man 4-Chlorcarbonyl-2,5-dichlor-thiazol der Formel (V)

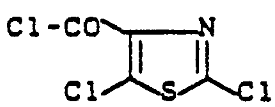

(V)

mit Ammoniak in Gegenwart eines inerten Verdünnungsmittels, wie z. B. Toluol, bei Temperaturen zwischen 0 °C und 40 °C umsetzt.

Das als Zwischenprodukt benötigte 4-Chlorcarbonyl-2,5-dichlor-thiazol der Formel (V) ist ebenfalls noch nicht aus der Literatur bekannt und Gegenstand der vorliegenden Erfindung.

Man erhält die neue Verbindung der Formel (V), wenn man 4-Carboxy-2,5-dichlor-thiazol der Formel (VI)

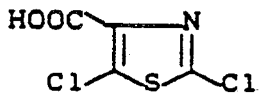

(VI)

mit einem Chlorierungsmittel, wie z. B. Thionylchlorid, bei Temperaturen zwischen 20 °C und 80 °C umsetzt.

Das als Zwischenprodukt benötigte 4-Carboxy-2,5-dichlorthiazol der Formel (VI) ist ebenfalls noch nicht aus der Literatur bekannt und Gegenstand der vorliegenden Erfindung.

Man erhält die neue Verbindung der Formel (VI), wenn man 2,5-Dichlor-4-trichlormethyl-thiazol der Formel (VII)

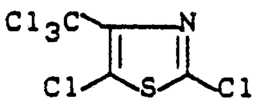

(VII)

mit Wasser bei Temperaturen zwischen 80 °C und 110 °C umsetzt.

Das als Zwischenprodukt benötigte 2,5-Dichlor-4-trichlormethyl-thiazol der Formel (VII) ist ebenfalls noch nicht aus der Literatur bekannt und Gegenstand der vorliegenden Erfindung.

Man erhält die neue Verbindung der Formel (VII), wenn man 2-Chlor-4-methyl-thiazol der Formel (VIII)

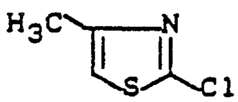

(VIII)

mit Chlor, gegebenenfalls in Gegenwart eines Verdünnungsmittels, wie z. B. Methylenchlorid, Chloroform oder Tetrachlormethan, bei Temperaturen zwischen 20 °C und 200 °C umsetzt.

2-Chlor-4-methyl-thiazol der Formel (VIII) ist bereits bekannt (vgl. J. Chem. Soc. 1919, 1071 - 1090).

Man erhält die Ausgangsverbindung der Formel (II) auf einfachere Weise, wenn man 2,5-Dichlor-4-trichlormethylthiazol der Formel (VII) - oben - mit Ammoniumchlorid in Gegenwart eines Katalysators, wie z. B. Eisen(III)chlorid, bei Temperaturen zwischen 150 °C und 250 °C umsetzt und das Produkt der Formel (II) durch Sublimation isoliert.

Die weiter als Ausgangsstoffe zu verwendenden Hydroxyessigsäureamide sind durch die Formel (III) allgemein definiert. In Formel (III) haben $R^1$ und $R^2$ vorzugsweise bzw. insbesondere diejenigen Bedeutungen, die bereits oben im Rahmen der Beschreibung der erfindungsgemäßen Verbindungen der Formel (I) als bevorzugt bzw. als insbesondere bevorzugt für $R^1$ bzw. $R^2$ angegeben wurden.

Beispiele für die Ausgangsstoffe der Formel (III) sind in der nachstehenden Tabelle 1 aufgeführt.

**Tabelle 1:** Beispiele für die Ausgangsstoffe der Formel (III)

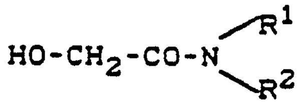

(III)

| $R^1$ | $R^2$ bzw. $-NR^1R^2$ | $R^1$ | $R^2$ bzw. $-NR^1R^2$ |
|---|---|---|---|
| $CH_3$ | $OCH(CH_3)_2$ | $CH_3$ | $OCH_2CH_2OC_2H_5$ |
| $CH_3$ | $CH(CH_3)_2$ | $CH_3$ | $CH_2CH(CH_3)_2$ |
| $CH_3$ | $CH-CH_2CH_2$<br>\|<br>$CH_3$ | $C_2H_5$ | $OC_2H_5$ |
| $C_2H_5$ | $OCH_2CH_2OC_2H_5$ | $C_2H_5$ | $OCH_2CH(CH_3)_2$ |
| $CH_3$ | 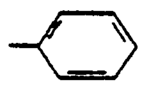 | $CH_3$ | 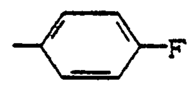 |
| $CH_3$ | 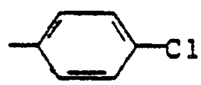 | $CH_3$ | 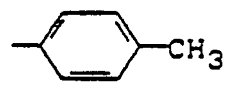 |
| $CH_3$ | 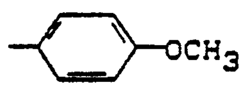 | $CH_3$ | 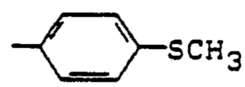 |
| $CH_3$ | 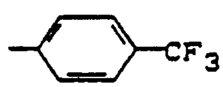 | $CH_3$ | 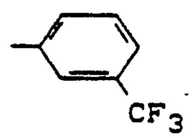 |
| $CH_3$ | 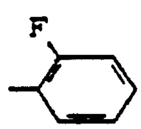 | $CH_3$ | 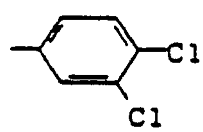 |

Tabelle 1 - Fortsetzung

| $R^1$ | $R^2$ bzw. $-N<^{R^1}_{R^2}$ |
|---|---|
| $CH_3$ | 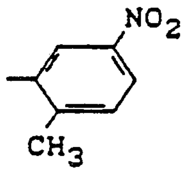 |
| $CH_3$ | 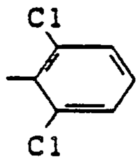 |
| $CH_3$ | 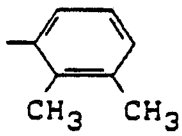 |
| $CH_3$ | 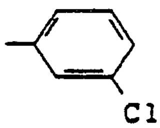 |
| $CH_3$ | 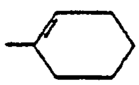 |
| $CH(CH_3)_2$ | 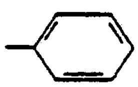 |
| $C_2H_5$ | 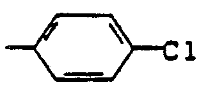 |
| $CH(CH_3)_2$ | 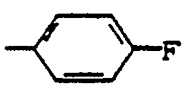 |
| $CH(CH_3)_2$ | 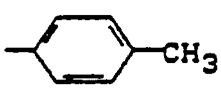 |
| $C_2H_5$ | 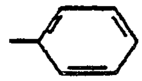 |
| $CH_3$ | 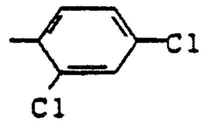 |
| $CH_3$ | 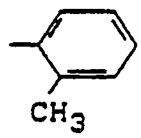 |
| $CH_3$ | 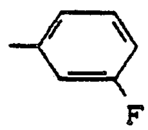 |
| $CH_3$ | $CH_3$ |
| $C_2H_5$ | 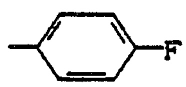 |
| $C_2H_5$ | 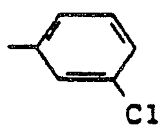 |
| $CH(CH_3)_2$ | 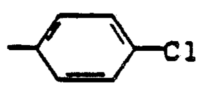 |
| $CH(CH_3)_2$ | 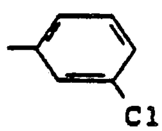 |

<u>Tabelle 1</u> - Fortsetzung

| $R^1$ | $R^2$ bzw. $-N\begin{smallmatrix}R^1\\R^2\end{smallmatrix}$ | $R^1$ | $R^2$ bzw. $-N\begin{smallmatrix}R^1\\R^2\end{smallmatrix}$ |
|---|---|---|---|
| $CH(CH_3)_2$ | 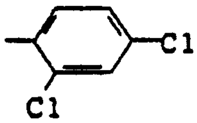 | $CH(CH_3)_2$ | 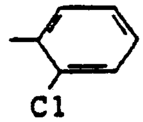 |
| $C_2H_5$ | $C_2H_5$ | $C_3H_7$ | $C_3H_7$ |
| $CH(CH_3)_2$ | $CH(CH_3)_2$ | $CH(CH_3)_2$ | $OCH_3$ |
| $CH(CH_3)_2$ | $OCH_2CH_2OC_2H_5$ | $CH(CH_3)_2$ | $OC_2H_5$ |
| $CH(CH_3)_2$ | $OC_3H_7$ | $CH_3$ | $OCH_2CH(CH_3)_2$ |
| $CH_3$ | $CH_2OCH_3$ | $C_4H_9$ | $C_4H_9$ |
| $C_2H_5$ | $OCH_3$ | $CH_3$ | $CH_2CF_3$ |
| $CH_2CH=CH_2$ | $CH_2CH=CH_2$ | $CH_2C{\equiv}CH$ | $CH_2C{\equiv}CH$ |
| $CH_3$ | $CH_2C{\equiv}CH$ | $CHCH_2CH_3$<br>\|<br>$CH_3$ | $OCH_3$ |
| $CHCH_2CH_3$<br>\|<br>$CH_3$ | $OCH_3$ | $CH_3$ | $CH_2$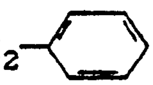 |
| $C_2H_5$ | $CH_2$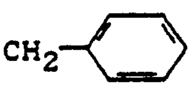 | $CH(CH_3)_2$ | $CH_2$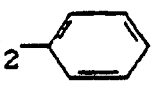 |
| $C_2H_5$ | $CH_2C{\equiv}CH$ | $CH(CH_3)_2$ | $CH_2C{\equiv}CH$ |
| $CH(CH_3)_2$ | $OCH(CH_3)_2$ | | |
| | 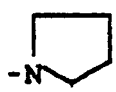 | | 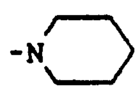 |
| | 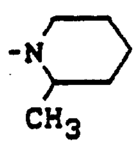 | | 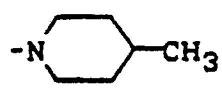 |

**Tabelle 1 - Fortsetzung**

| $R^1$ | $R^2$ bzw. $-N{<}^{R^1}_{R^2}$ | $R^1$ | $R^2$ bzw. $-N{<}^{R^1}_{R^2}$ |
|---|---|---|---|
| | 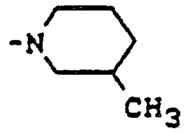 | | 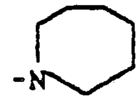 |
| | 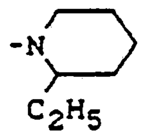 | | 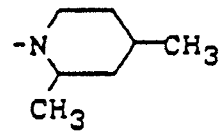 |
| | 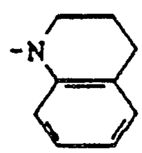 | | |

Die neuen Zwischenprodukte der Formeln (II), (IV), (V), (VI) und (VIII) können auch zusammenfassend beschrieben werden als 4-substituierte 2,5-Dichlor-thiazol-Derivate der Formel (IX)

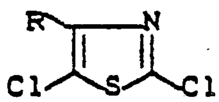

(IX)

worin
R für $-C{\equiv}N$, $-CONH_2$, $-COCl$, $-COOH$ oder $-CCl_3$ steht.

Die Hydroxyessigsäureamide der Formel (II) sind bekannt und/oder können nach an sich bekannten Verfahren hergestellt werden (vgl. EP-A 37 526, US-P 4 509 971, US-P 4 645 525, US-P 4 334 073).

Das erfindungsgemäße Verfahren zur Herstellung der neuen 5-Chlor-4-cyano-thiazol-2-yl-oxyessigsäureamide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid. Besonders bevorzugt ist Natriumchlorid.

Das erfindungsgemäße Verfahren wird vorteilhaft unter Verwendung von Säurebindemitteln durchgeführt. Als sol che werden vorzugsweise stark basische Alkali- und Erdalkalimetallverbindungen, beispielsweise Oxide, wie z. B. Natrium-, Kalium, Magnesium- und Calciumoxid, Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:

Tetrabutylammoniumchlorid, Tetrabutylammoniumbromid, Tributyl-methylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +100 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol 4-Cyano-2,5-dichlor-thiazol der Formel (II) im allgemeinen zwischen 0,5 und 5 Mol, vorzugsweise zwischen 0,8 und 1,5 Mol, Hydroxyessigsäureamid der Formel (III) ein. Die Reaktionskomponenten können in beliebiger Reihenfolge zusammen gegeben weden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgernaßen Verbindungen der Formel (I) eignen sich insbesondere zur selektiven Bekämpfung von monokotylen Unkräutern in dikotylen Kulturen, vor allem im Vorauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farb stoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 5-Amino-4-chlor-2-phenyl-2,3-dihydro-3-oxy-pyridazin (CHLORIDAZON); Ethyl-2-{[(4-chlor-6-methoxy-2-pyrimidinyl)-aminocarbonyl]-aminosulfonyl}-benzoat (CHLORIMURON); N,S-Diethyl-N-cyclohexyl-thiolcarbamat (CYCLOATE); 2-[(2-Chlorphenyl)-methyl]-4,4-dimethyl-isoxazolidin-3-on (DIMETHAZONE); 4-Amino-6-t-butyl-3-ethylthio-1,2,4-triazin-5(4H)-on (ETHIOZIN); N,N-Dimethyl-N′-(3-trifluormethylphenyl)-harnstoff (FLUOMETURON); 5-(2-Chlor-4-trifluormethyl-phenoxy)-N-methylsulfonyl-2-nitrobenzamid (FOMESAFEN); 2-[5-Methyl-5-(1-methylethyl)-4-oxo-2-imidazolin-2-yl]-3-chinolincarbonsäure (IMAZAQUIN); 2-[4,5-Dihydro-4-methyl-4-isopropyl-5-oxo-(1H)-imidazol-2-yl]-5-ethyl-pyridin-3-carbonsäure (IMAZETHAPYR); 1-(3-Trifluormethyl-phenyl)-4-methylamino-5-chlor-6-pyridazon (NORFLURAZON); N-(1-Ethylpropyl)-3,4-dimethyl-2,6-dinitroanilin (PENDIMETHALIN); 3-[[[[(4-Methoxy-6-methyl-1,3,5-triazin-2- yl)-amino]-carbonyl]-amino]-sulfonyl]-thiophen-2-carbonsäure-methylester (THIAMETURON) und 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hek tar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

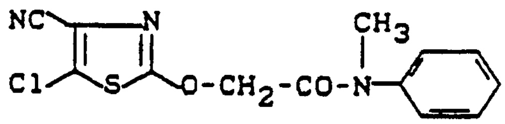

Eine Lösung von 8,9 g (0,05 Mol) 4-Cyano-2,5-dichlorthiazol in 15 ml Acetonitril wird unter Rühren zu einer auf -20 °C abgekühlten Mischung aus 8,2 g (0,05 Mol) Hydroxyessigsäure-N-methyl-anilid, 3,1 g (0,05 Mol) Kaliumhydroxid und 90 ml Isopropanol tropfenweise gegeben. Das Reaktionsgemisch wird 5 Stunden bei -15 °C bis -20 °C und 12 Stunden im Eis-Kochsalz-Bad gerührt, dann mit Essigsäure angesäuert und im Wasserstrahlvakuum eingeengt. Der Rückstand wird mit Wasser verrührt, wobei sich das Produkt kristallin abscheidet, welches durch Absaugen isoliert wird.

Man erhält 12 g (80 % der Theorie) 2-(5-Chlor-4-cyanothiazol-2-yl-oxy)-essigsäure-N-methyl-anilid vom Schmelzpunkt 80 °C.

Analog Beispiel 1 und entsprechend der allgemeinen Beschreibung des erfindungsgemäßen Herstellungsverfahrens können die in der nachstehenden Tabelle 2 aufgeführten Verbindungen der Formel (I) hergestellt werden:

**Tabelle 2: Beispiele für die Verbindungen der Formel (I)**

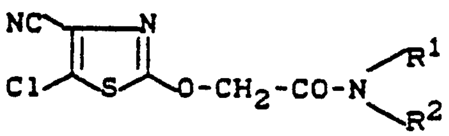

(I)

Tabelle 2

| Beisp.-Nr. | $R^1$ | $R^2$ | Physikalische Daten |
|---|---|---|---|
| | bzw. 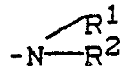 | | |
| 2 | $CH(CH_3)_2$ | $OCH_2CH_2OC_2H_5$ | $n_D^{20}$: 1,5101 |
| 3 | $CH_3$ | $OCH_3$ | Fp.: 105 °C |
| 4 | $CH(CH_3)_2$ | 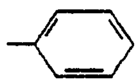 | Fp.: 76 °C |
| 5 | $CH_3$ | 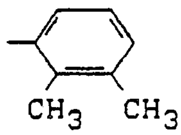 | Fp.: 84 °C |
| 6 | $CH_2CH=CH_2$ | $CH_2CH=CH_2$ | $n_D^{20}$: 1,5430 |
| 7 | 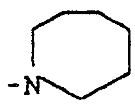 | | $n_D^{20}$: 1,5594 |
| 8 | $C_2H_5$ | $C_2H_5$ | $n_D^{20}$: 1,5438 |
| 9 | $CH(CH_3)_2$ | 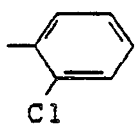 | Fp.: 74 °C |
| 10 | $CH(CH_3)_2$ | 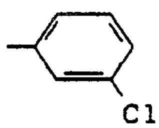 | Fp.: 61 °C |
| 11 | $CH(CH_3)_2$ | 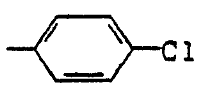 | Fp.: 72 °C |

<u>Tabelle 2</u> - Fortsetzung

| Beisp.-Nr. | $R^1$ \| $R^2$ bzw. $-N<^{R^1}_{R^2}$ | | Physikalische Daten |
|---|---|---|---|
| 12 | $CH_3$ | 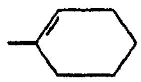 | Fp.: 98 °C |
| 13 | $CH_3$ | 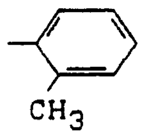 | Fp.: 96 °C |
| 14 | $CH_3$ | 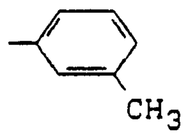 | Fp.: 87 °C |
| 15 | $CH_3$ | 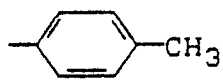 | |
| 16 | $CH_3$ | 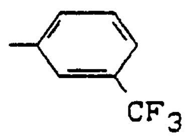 | |
| 17 | $CH_3$ | 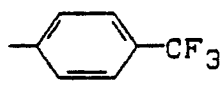 | |
| 18 | $C_3H_7$ | $C_3H_7$ | |
| 19 | 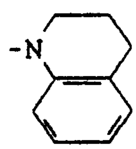 | | Fp.: 70 °C |
| 20 | $CH_3$ | $C_4H_9$ | $n_D^{20}$ : 1,4289 |
| 21 | $CHC_2H_5$<br>\|<br>$CH_3$ | $OCH_3$ | |

Tabelle 2 - Fortsetzung

| Beisp.-Nr. | $R^1$ | $R^2$ bzw. 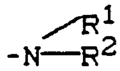 | Physikalische Daten |
|---|---|---|---|
| 22 | $CH_3$ | $CH_2C{\equiv}CH$ | |
| 23 | $CH_3$ | 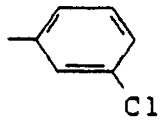 | Fp.: 77 °C |
| 24 | $CH_3$ | 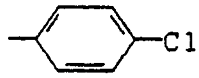 | Fp.: 110 °C |
| 25 | $C_4H_9$ | $C_4H_9$ | Fp.: 38 °C |
| 26 | 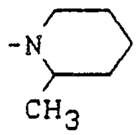 | | |
| 27 | 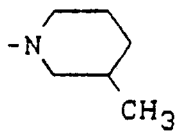 | | $n_D^{20}$: 1,5419 |
| 28 | 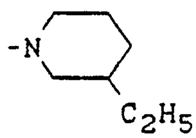 | | |
| 29 | $CH_3$ | $CH_2OCH_3$ | Fp.: 64 °C |
| 30 | 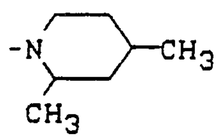 | | |
| 31 | $CH(CH_3)_2$ | 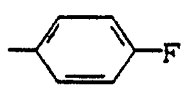 | |

Tabelle 2 - Fortsetzung

| Beisp.-Nr. | $R^1$ | $R^2$ bzw. $-N<^{R^1}_{R^2}$ | Physikalische Daten |
|---|---|---|---|
| 32 | $CH(CH_3)_2$ | 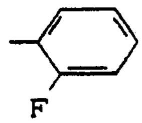 | |
| 33 | $CH(CH_3)_2$ | 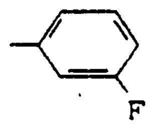 | |
| 34 | | 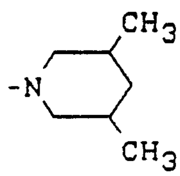 | $n_D^{20}$ : 1,5329 |

Ausgangsverbindung der Formel (II)

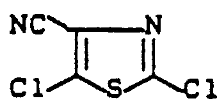 (II)

127,8 g (0,649 Mol) 2,5-Dichlor-thiazol-4-carboxamid und 1300 ml Phosphoroxychlorid wurden unter Rühren etwa 5 Stunden unter Rückfluß (ca. 110 °C) erhitzt. Nach dem Abdestillieren der Hauptmenge überschüssigen Phosphoroxychlorids im Wasserstrahlvakuum wurde der ölige Rückstand tropfenweise zu etwa 1 l Wasser gegeben, das durch Kühlung zwischen 15 °C und 20 °C gehalten wurde. Der dabei entstehende kristalline Niederschlag wurde abfiltriert, mit Wasser gewaschen und getrocknet.

Man erhielt so 103 g (88,7 % der Theorie) 4-Cyano-2,5-dichlorthiazol. Die Verbindung ist bei 114 °C/16 mbar destillierbar, bei 80 °C/16 mbar sublimierbar und schmilzt bei 56,5 °C - 57 °C.

Alternativmethode:

Eine Mischung aus 5,43 g (20 mmol) 2,5-Dichlor-4-trichlormethyl-thiazol, 2,14 g (40 mmol) Ammoniumchlorid und 0,27 g wasserfreiem Eisen(III)-chlorid wurde unter trocknem Stickstoff unter gutem Rühren 2 Stunden auf 200 °C erhitzt. Danach wurden bis 200 °C/0,1 mbar alle sublimierbaren Anteile aus dem Reaktionsgemisch absublimiert und dieses Rohsublimat bis 85 °C/0,1 mbar resublimiert.

Man erhielt so 2,0 g (55,9 % der Theorie) reines 4-Cyano-2,5-dichlorthiazol, das in allen Eigenschaften mit dem 4-Cyano-2,5-dichlorthiazol aus dem vorigen Beispiel identisch ist.

Ausgangsverbindung der Formel (IV)

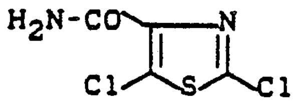

(IV)

Über eine Lösung von 144,6 g (0,668 Mol) 4-Chlorcarbonyl-2,5-dichlor-thiazol in 750 ml wasserfreiem Toluol wurden unter Rühren und Kühlung bei maximal 20 °C 61 g (3,59 Mol) Ammoniakgas geleitet. Anschließend wurde der entstandene Niederschlag abfiltriert, mit Toluol gewaschen, getrocknet und etwa eine Stunde in 1 l Eiswasser verrührt.

Nach dem Abfiltrieren, Waschen mit Wasser und Trocknen wurden 127,9 g (97,2 % der Theorie) 2,5-Dichlor-thiazol-4-carboxamid vom Schmelzpunkt 153 °C erhalten.

Ausgangsverbindung der Formel (V)

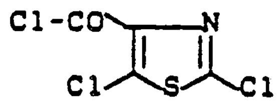

(V)

Eine Mischung aus 143,5 g (0,725 Mol) 2,5-Dichlor-thiazol-4-carbonsäure und 700 ml Thionylchlorid wurde unter Rühren langsam erhitzt. Bereits bei etwa 40 °C trat starke Gasentwicklung ein. Im Verlauf einer halben Stunde wurde bis auf Rückflußtemperatur erhitzt und dort bis zum Ende der Gasentwicklung gehalten (ca. 2 Stunden): Endtemperatur etwa 80 °C.

Nach dem Abziehen des überschüssigen Thionylchlorids im Wasserstrahlvakuum hinterblieben 144,6 g (92,2 % der Theorie) kristallines 4-Chlorcarbonyl-2,5-dichlor-thiazol. Aus Petrolether große, farblose Kristalle vom Schmelzpunkt 58 °C - 59 °C.

Ausgangsverbindung der Formel (VI)

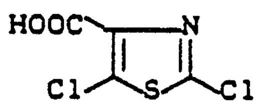

(VI)

271,5 g (1 Mol) 2,5-Dichlor-4-trichlormethyl-thiazol und 2700 ml Wasser wurden unter Rühren über Nacht (etwa 15 Stunden) unter Rückfluß erhitzt. Nach dem Abkühlen auf Raumtemperatur wurde der entstandene kristalline Niederschlag abfiltriert, mit Wasser gewaschen und getrocknet.

Ausbeute 143,5 g (72,5 % der Theorie) 2,5-Dichlor-thiazol-4-carbonsäure. Die Verbindung ist bei 120 °C/0,1 mbar sublimierbar und z. B. aus Chloroform umkristallisierbar. Schmelzpunkt 191 °C unter Zersetzung. Durch Einengen der wäßrigen Phase konnten weitere 30,5 g etwas weniger reines Produkt isoliert werden.

Ausgangsverbindung der Formel (VII)

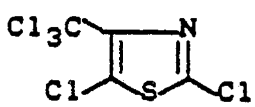

(VII)

In einem Dreihalskolben, der mit Rührer, Thermometer, Rückflußkühler und Gaseinleitungsrohr versehen war, wurde, beginnend bei Raumtemperatur, in eine Mischung aus 1093 g (8,19 Mol) 2-Chlor-4-methyl-thiazol und 4 l Methylenchlorid Chlorgas eingeleitet. Nach Abklingen der exothermen Reaktion wurde unter allmählicher Temperatursteigerung und weiterem Einleiten von Chlor zunächst das Methylenchlorid abdestilliert und dann der Sumpf langsam bis auf etwa 160 °C erhitzt. Bei etwa 160 °C wurde dann so lange

meist überschüssiges Chlorgas (erkennbar an der leicht grünlichen Farbe des Abgases) eingeleitet, bis im Gaschromatogramm fast nur noch die gewünschte Verbindung 2,5-Dichlor-4-trichlormethyl-thiazol zu erkennen war. Gesamtdauer der Chlorierung 40 - 50 Stunden.

Eine Grobdestillation bis zu einer Kopftemperatur von 150 °C bei 14 mbar erbrachte 2057 g ca. 95 %iges 2,5-Dichlor-4-trichlormethyl-thiazol, was einer Ausbeute von 88 % der Theorie, bezogen auf reines Podukt, entspricht. Das 2,5-Dichlor-4-trichlormethyl-thiazol wurde durch eine Feindestillation an einer ca. 220 cm langen, silberverspiegelten Füllkörperkolonne rein erhalten. Siedepunkt 123 °C - 125 °C bei 16 mbar.

Verwendungsbeispiele

In den folgenden Verwendungsbeispielen wird die Verbindung nachstehender Formel als Vergleichssubstanz eingesetzt:

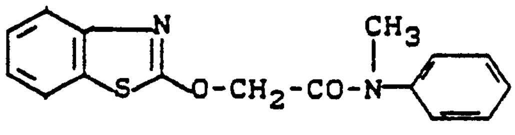

(A)

2-(Benzthiazol-2-yl-oxy)-essigsäure-N-methyl-anilid
(bekannt aus US-P 4 509 071).

Beispiel A

Pre-emergence-Test

| | |
|---|---|
| Lösungsmittel: | 5 Gewichtsteile Aoeton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

| | | |
|---|---|---|
| 0 % | = | keine Wirkung (wie unbehandelte Kontrolle) |
| 100 % | = | totale Vernichtung |

Eine deutliche Überlegenheit in der Wirksamkeit gegenüber dem Stand der Technik zeigen in diesem Test z.B. die Verbindungen gemäß den Herstellungsbeispielen (1), (2), (4), (5), (6), (7) und (8).

## Ansprüche

1. 5-Chlor-4-cyano-thiazol-2-yl-oxyessigsäureamide der allgemeinen Formel (I)

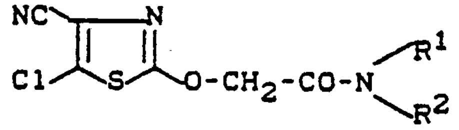

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Heterocyclus bilden, der weitere Heteroatome enthalten kann und an den eine Benzo-Gruppierung anelliert sein kann.

2. Verbindungen der allgemeinen Formel (I) gemäß Anspruch 1, dadurch gekennzeichnet, daß darin

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$-oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

3. Verbindungen der allgemeinen Formel (I) gemäß Anspruch 1, dadurch gekennzeichnet, daß darin

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy, Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein-oder zweifach durch Methyl und/oder Ethyl substituiertes Pyrrolidinyl, für Perhydroazepinyl oder für 1,2,3,4-Tetrahydro-chinolinyl stehen.

4. Verfahren zur Herstellung von 5-Chlor-4-cyanothiazol-2-yl-oxyessigsäureamiden der Formel (I) gemäß Anspruch 1, dadurch gekennzeichnet, daß man 4-Cyano-2,5-dichlor-thiazol der Formel (II)

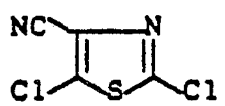

(II)

mit Hydroxyessigsäureamiden der allgemeinen Formel (III)

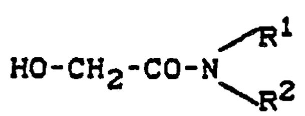

(III)

in welcher

$R^1$ und $R^2$ die in Anspruch 1 angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

5. Herbizide Mittel, gekennzeichnet durch einen Gehalt an mindestens einem 5-Chlor-4-cyano-thiazol-2-yl-oxyessigsäureamid der Formel (I) gemäß Anspruch 1.

6. Verwendung von 5-Chlor-4-cyano-thiazol-2-yl-oxyessigsäureamiden der Formel (I) gemäß Anspruch 1 zur Bekämpfung von Pflanzenwachstum.

7. Verfahren zur Herstellung von herbiziden Mitteln, dadurch gekennzeichnet, daß man 5-Chlor-4-cyano-thiazol-2-yl-oxyessigsäureamide der Formel (I) gemäß Anspruch 1 mit Streckmitteln und/oder oberflächenaktiven Mitteln vermischt.

8. 4-Substituierte 2,5-Dichlor-thiazol-Derivate der Formel (IX)

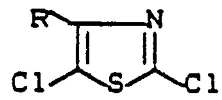

(IX)

worin
R für $-C\equiv N$, $-CONH_2$, $-COCl$, $-COOH$ oder $-CCl_3$ steht.